# EUROPÄISCHE PATENTANMELDUNG

(11) **EP 3 229 014 A2**
(43) Veröffentlichungstag der Anmeldung: **11.10.2017**
(21) Anmeldenummer: 17163403.3
(22) Anmeldetag: 07.05.2014
(51) Int. Cl.: G01N 21/94, G01N 21/64, G01N 33/12, G01N 21/84

(54) **MESSGERÄT ZUM MESSEN EINES OBERFLÄCHENBELAGS AUF EINEM MESSOBJEKT, NÄMLICH AUF EINEM LEBENSMITTEL**

(30) Priorität: 08.05.2013 DE 102013008003
(62) Teilanmeldung aus: 14725632.5
(71) Anmelder: FreshDetect GmbH, 82049 Pullach i. Isartal (DE)
(72) Erfinder: Reichl, Mathias, 93309 Kelheim (DE); Klein, Rolf-Dieter, 80809 München (DE)
(74) Vertreter: v. Bezold & Partner Patentanwälte - PartG mbB

(57) **Zusammenfassung**

Die Erfindung betrifft ein Messgerät (1; 30) zum Messen eines Oberflächenbelags (18) auf einem Messobjekt (19; 24), nämlich auf einem Lebensmittel, insbesondere zum Messen von Stoffwechselprodukten von Bakterien auf Fleisch, das zum Verzehr bestimmt ist, mit mindestens einer Anregungsquelle (3-6) zur Lumineszenzanregung des Oberflächenbelags (18) auf dem Messobjekt (19; 24), so dass der Oberflächenbelag (18) eine Lumineszenzstrahlung abgibt, und mit mindestens einem optischen Sensor (10) zur Erfassung der Lumineszenzstrahlung, die von dem Oberflächenbelag (18) abgegeben wird.

## Beschreibung

Die Erfindung betrifft ein Messgerät zum Messen eines Oberflächenbelags auf einem Messobjekt (z.B. Lebensmittel), insbesondere zum Messen von Stoffwechselprodukten von Bakterien auf Fleisch, das zum Verzehr bestimmt ist.

Ein derartiges Messgerät ist unter der Bezeichnung "Fresh Scan" aus einem Forschungsprojekt bekannt. Dieses bekannte Messgerät beruht auf der Erkenntnis, dass Fleisch während der Lagerung zunehmend von Bakterien besiedelt wird, deren Stoffwechselprodukte (z.B. Porphyrine) fluoreszieren, so dass die Messung der von den Stoffwechselprodukten der Bakterien abgegebenen Fluoreszenzstrahlung einen Rückschluss auf den Frischezustand und die Gesamtkeimzahl (GKZ)des Fleischs erlaubt.

Das bekannte Messgerät weist zur Anregung der Fluoreszenzstrahlung einen Laser auf, dessen Strahlung auf das zu prüfende Fleisch gerichtet wird, so dass die Stoffwechselprodukte der Bakterien auf dem Fleisch Fluoreszenzstrahlung aussenden, die dann von einem optischen Sensor erfasst wird, wobei der optische Sensor in der Fluoreszenzstrahlung bestimmte Wellenlängen abtastet, die für die Fluoreszenzstrahlung der Stoffwechselprodukte der Bakterien charakteristisch sind.

Zum einen berücksichtigt das bekannte Messgerät also nicht das gesamte Spektrum der Fluoreszenzstrahlung, sondern lediglich bestimmte charakteristische Wellenlängen der Fluoreszenzstrahlung.

Zum anderen ist zu erwähnen, dass das bekannte Messgerät die Fluoreszenzstrahlung lediglich an einem einzigen Messpunkt misst.

Im Betrieb des vorstehend beschriebenen bekannten Messgeräts kommt es deshalb gelegentlich zu Fehlmessungen.

Weiterhin ist zum Stand der Technik hinzuweisen auf DE 10 2011 100 507 A1, DE 27 28 717 A1, US 6 597 932 B2, WO 97/08538 A1, WO 99/57529 A1, WO 2007/079943 A1, EP 0 128 889 B2, DE 602 08 823 T2, US 5 760 406 A, US 5 914 247 A, AT 414 275 B, DE 103 15 541 A1, DE 10 2005 051 643 A1, WO 2003/070080 A1, US 5 658 798 A1, US 5 474 910 A, EP 1 329 514 A2, US 4 866 283 A, DE 44 20 401 A1 und US 6 649 412 B1. Diese Druckschriften offenbaren jedoch lediglich Messgeräte bzw. Messprinzipien, die nicht optimal sind.

Der Erfindung liegt deshalb die Aufgabe zugrunde, das eingangs beschriebene bekannte Messgerät zu verbessern.

Diese Aufgabe wird durch ein erfindungsgemäßes Messgerät gemäß dem Hauptanspruch gelöst.

Das erfindungsgemäße Messgerät weist in Übereinstimmung mit dem Stand der Technik mindestens eine Anregungsquelle auf, um in dem Oberflächenbelag des zu überwachenden Lebensmittels Lumineszenz anzuregen, so dass der Oberflächenbelag eine Lumineszenzstrahlung abgibt.

In einem bevorzugten Ausführungsbeispiel der Erfindung ist die Anregungsquelle - wie bei dem bekannten Messgerät - eine Lichtquelle, so dass die von der Lichtquelle angeregte Lumineszenzstrahlung eine Photolumineszenzstrahlung ist, insbesondere eine Fluoreszenzstrahlung oder eine Phosphoreszenzstrahlung. Die Erfindung ist jedoch hinsichtlich der Anregung der Lumineszenzstrahlung nicht auf eine optische Anregung beschränkt. Vielmehr ist es im Rahmen der Erfindung auch grundsätzlich denkbar, andere Arten von Lumineszenz auszunutzen, wie beispielsweise Elektrolumineszenz, Kathodolumineszenz, Chemolumineszenz, Biolumineszenz, Tribolumineszenz, Thermolumineszenz, Sonolumineszenz, Radiolumineszenz, Ionolumineszenz oder Piezolumineszenz. Hierbei kann auch die Abklingzeit der Fluoreszenz ausgewertet werden.

In dem bevorzugten Ausführungsbeispiel der Erfindung mit einer Lichtquelle zur Photolumineszenzanregung strahlt die Lichtquelle vorzugsweise ein Spektrum ab, das einen Wellenlängenbereich von 350nm-550nm aufweist. In einem bevorzugten Ausführungsbeispiel strahlt die Lichtquelle eine Welleänge von 405nm ab.

Beispielsweise kann es sich bei der Lichtquelle um eine Laserdiode oder um eine Leuchtdiode handeln, jedoch besteht grundsätzlich auch die Möglichkeit der Verwendung einer anderen Lichtquelle, wie beispielsweise einer Glühlampe.

Darüber hinaus weist das erfindungsgemäße Messgerät in Übereinstimmung mit dem eingangs beschriebenen bekannten Messgerät mindestens einen optischen Sensor auf, um die Lumineszenzstrahlung zu messen, die von dem Oberflächenbelag auf dem Lebensmittel abgegeben wird.

In dem bevorzugten Ausführungsbeispiel misst der optische Sensor die Lumineszenzstrahlung von allen Messpunkten, wobei die Messung an den einzelnen Messpunkten beispielsweise zeitsequenziell erfolgen kann.

Es besteht jedoch alternativ auch die Möglichkeit, dass jedem Messpunkt jeweils ein eigener optischer Sensor zugeordnet ist, der die Lumineszenzstrahlung an dem jeweiligen Messpunkt misst.

Bei der Verwendung eines einzigen optischen Sensors zur Messung an allen Messpunkten weist der optische Sensor vorzugsweise einen so großen Messwinkel auf, dass alle Messpunkte innerhalb des Messwinkels liegen, so dass der optische Sensor die Lumineszenzstrahlung von allen Messpunkten messen kann.

Beispielsweise kann die von dem Oberflächenbelag abgegebene Lumineszenzstrahlung über einen Lichtleiter mit geeigneter numerischer Aperatur (NA) zu dem optischen Sensor geleitet werden.

Weiterhin ist zu erwähnen, dass der optische Sensor vorzugsweise ein Spektralphotometer ist, das ein Wellenlängenspektrum der Lumineszenzstrahlung des Oberflächenbelags misst. Dadurch unterscheidet sich das erfindungsgemäße Messgerät von dem eingangs beschriebenen bekannten Messgerät, bei dem lediglich die Intensität der Lumineszenzstrahlung an bestimmten charakteristischen Wellenlängen gemessen wird, wohingegen das Spektralphotometer das komplette Wellenlängenspektrum der Lumineszenzstrahlung erfasst. Dies ist vorteilhaft, weil dadurch charakteristische Wellenlängenspektren der Lumineszenzstrahlung erfasst werden können, wodurch Fehlmessungen weitgehend vermieden werden können. Im Rahmen der Erfindung besteht also auch die Möglichkeit einer Signalauswertung durch das sogenannte "Spectral Imaging", was an sich aus dem Stand der Technik bekannt ist.

Ferner ist zu erwähnen, dass vorzugsweise mehrere Messpunkte vorgesehen sind, wobei die einzelnen Messpunkte vorzugsweise um die optische Achse des optischen Sensors verteilt angeordnet sind. Dies ist vorteilhaft, weil der optische Sensor dann leichter die Lumineszenzstrahlung von allen Messpunkten erfassen kann.

In dem bevorzugten Ausführungsbeispiel der Erfindung berücksichtigt das Messgerät nicht nur das Wellenlängenspektrum der Lumineszenzstrahlung, die von dem Oberflächenbelag abgegeben wird, sondern auch die Oberflächentemperatur des Oberflächenbelags auf dem zu überwachenden Lebensmittel. Hierzu weist das erfindungsgemäße Messgerät vorzugsweise ein Pyrometer auf, das eine berührungslose Temperaturmessung ermöglicht.

Ferner enthält das erfindungsgemäße Messgerät vorzugsweise auch ein Kolorimeter zur Messung der Farbe des Oberflächenbelags ohne die Lumineszenzanregung, wobei derartige Kolorimeter an sich aus dem Stand der Technik bekannt sind und deshalb nicht näher beschrieben werden müssen.

Die Messgenauigkeit lässt sich im Rahmen der Erfindung weiter verbessern, wenn auch das zeitliche Abklingverhalten der Lumineszenzstrahlung des Oberflächenbelags berücksichtigt wird. Das erfindungsgemäße Messgerät weist deshalb vorzugsweise ein Messglied auf, welches das zeitliche Abklingverhalten der Lumineszenzstrahlung misst.

Darüber hinaus kann das erfindungsgemäße Messgerät auch ein pH-Meter aufweisen, um den pH-Wert des Oberflächenbelags auf dem Lebensmittel zu messen.

Weiterhin kann das erfindungsgemäße Messgerät eine Widerstandsmesseinrichtung aufweisen, um den elektrischen Oberflächenwiderstand des Oberflächenbelags und/oder des Lebensmittels zu messen, wodurch die Messgenauigkeit weiter verbessert werden kann.

Ferner ist zu erwähnen, dass das erfindungsgemäße Messgerät eine Auswertungseinheit zur Qualifizierung und/oder zur Quantifizierung des Oberflächenbelags und/oder des Lebensmittels und zur Erzeugung eines entsprechenden Ausgangssignals enthält. Das Ausgangssignal der Auswertungseinheit kann beispielsweise eine der folgenden Eigenschaften des zu prüfenden Lebensmittels wiedergeben:
- Keimzahl von Stoffwechselprodukten von Bakterien in dem Oberflächenbelag, insbesondere von Porphyrinen, insbesondere von Protoporphyrin IX.
- Typ des Lebensmittels aus einer Gruppe von Fleisch, Fisch, Gemüse und/oder Obst.
- Typ des Fleischs aus einer Gruppe von Schweinefleisch, Rindfleisch, Geflügelfleisch, Lammfleisch, Wildfleisch, Pferdefleisch und/oder Hundefleisch.
- Verzehrbarkeit des Lebensmittels in Abhängigkeit vom Frischezustand des Lebensmittels.

Es wurde bereits vorstehend darauf hingewiesen, dass Fleisch während der Lagerung zunehmend von Bakterien besiedelt wird, deren Stoffwechselprodukte eine charakteristische Fluoreszenzstrahlung abstrahlen, so dass die Messung der Fluoreszenzstrahlung einen Rückschluss auf den Frischezustand des Fleischs ermöglicht. Bei der Auswertung der gemessenen Fluoreszenzstrahlung soll nach Möglichkeit nur selektiv das charakteristische Wellenlängenspektrum berücksichtigt werden, das von den jeweiligen bakteriellen Stoffwechselprodukten (z.B. Porphyrine) emittiert wird. Die Auswertungseinheit misst deshalb bei den Peaks der Lumineszenzstrahlung die Wellenlänge des jeweiligen Peaks und die Intensität des jeweiligen Peaks. Anschließend ermittelt die Auswertungseinheit dann vorzugsweise das Intensitätsverhältnis der Peaks, also beispielsweise das Verhältnis der Intensität des ersten Peaks und der Intensität des zweiten Peaks. Weiterhin ermittelt die Auswertungseinheit vorzugsweise die Wellenlängenübereinstimmung zwischen den Wellenlängen der gemessenen Peaks der Lumineszenzstrahlung einerseits und vorgegebenen charakteristischen Wellenlängen andererseits, die für die Fluoreszenzstrahlung der bakteriellen Stoffwechselprodukte charakteristisch sind. Beispielsweise können die charakteristischen Wellenlängen die Wellenlängen von Peaks in der Fluoreszenzstrahlung von Porphyrinen sein, insbesondere von Protoporphyrin IX. Das Intensitätsverhältnis der gemessenen Peaks und die Wellenlängenübereinstimmung der gemessenen Peaks mit den charakteristischen Wellenlängen ermöglichen dann eine Beurteilung, ob die gemessene Strahlung tatsächlich von bakteriellen Stoffwechselprodukten herrührt oder auf Störungen beruht.

Weiterhin kann die Auswertungseinheit die Signalform der Peaks auswerten, um zu beurteilen, ob die detektierte Fluoreszenzstrahlung von dem Oberflächenbelag erzeugt wird oder durch Störungen.

Die Auswertungseinheit kann den Oberflächenbelag auf dem Lebensmittel (z.B. Fleisch) dann in Abhängigkeit von mindestens einer der folgenden Größen qualifizieren und/oder quantifizieren, wobei dann ein entsprechendes Ausgangssignal erzeugt wird:
- Gesamtwert, der die Intensitäten der Lumineszenzstrahlungen an allen Messpunkten wiedergibt, z.B. Mittelwert der gemessenen Intensitäten an den einzelnen Messpunkten,
- Intensitäten an den einzelnen Messpunkten,
- Oberflächentemperatur des Lebensmittels,
- Farbe des Oberflächenbelags auf dem Lebensmittel,
- Zeitliches Abklingverhalten der Lumineszenzstrahlung, die von dem Oberflächenbelag auf dem Lebensmittel abgestrahlt wird,
- Intensitätsverhältnis der Peaks der Lumineszenzstrahlung,
- Wellenlängenübereinstimmung zwischen den Peaks der Lumineszenzstrahlung und den vorgegebenen charakteristischen Wellenlängen,
- pH-Wert,
- Oberflächenwiderstand.

In dem bevorzugten Ausführungsbeispiel der Erfindung erfolgt die Auswertung der vorstehend genannten Eingangsgrößen durch eine Fuzzy-Logik, was an sich aus dem Stand der Technik bekannt ist und deshalb nicht näher beschrieben werden muss.

Ferner ist zu erwähnen, dass das erfindungsgemäße Messgerät vorzugsweise tragbar ist, beispielsweise in Form eines Handgeräts. Dies ermöglicht einen Einsatz beispielsweise in der Gastronomie oder in fleischverarbeitenden Betrieben.

Die Stromversorgung des erfindungsgemäßen Messgeräts kann beispielsweise durch eine integrierte Batterie erfolgen, wobei es sich vorzugsweise um eine wiederaufladbare Batterie handelt.

Darüber hinaus weist das erfindungsgemäße Messgerät vorzugsweise eine Anzeige auf, um das Ausgangssignal der Auswertungseinheit oder sonstige Betriebsparameter des Messgeräts anzeigen zu können. Beispielsweise kann es sich bei der Anzeige um eine LCD-Anzeige (LCD: Liquid Crystal Display) handeln.

Von Bedeutung ist weiterhin, dass das erfindungsgemäße Messgerät eine schnelle Messung ermöglicht, so dass das Messgerät auch in Fertigungsstraßen in fleischverarbeitenden Betrieben zum Einsatz kommen kann, ohne dass die Verarbeitungsgeschwindigkeit beeinträchtigt wird. Die Messdauer ist deshalb vorzugsweise kleiner als 10 Sekunden, 1 Sekunde oder 50 Millisekunden.

Darüber hinaus weist das erfindungsgemäße Messgerät vorzugsweise eine Datenschnittstelle auf, um das Messgerät zu konfigurieren und/oder um Messdaten auszugeben. Beispielsweise kann die Datenschnittstelle eine USB-Schnittstelle (USB: Universal Serial Bus), eine Bluetooth-Schnittstelle, eine WLAN-Schnittstelle (WLAN: Wireless Local Area Network) und/oder eine RFID-Schnittstelle (RFID: RadioFrequency-Identification) aufweisen.

In dem bevorzugten Ausführungsbeispiel der Erfindung weist das Messgerät eine durchsichtige und auswechselbare Kappe auf, wobei die Anregung der Lumineszenzstrahlung und die Messung der Lumineszenzstrahlung durch die Kappe hindurch erfolgen. Beispielsweise kann die Kappe einfach auf einen Messkopf des Messgeräts aufgesteckt bzw. abgezogen werden.

Weiter ist zu erwähnen, dass jedem Messpunkt vorzugsweise jeweils eine eigene Anregungsquelle zugeordnet ist, so dass die Lumineszenzanregung an jedem Messpunkt jeweils durch die diesem Messpunkt zugeordnete Anregungsquelle erfolgt. Es ist jedoch alternativ auch möglich, dass nur eine einzige Anregungsquelle vorgesehen ist, welche die Lumineszenzanregung an sämtlichen Messpunkten bewirkt.

Denkbar ist im Rahmen der Erfindung auch die Kombination der vorstehenden beschriebenen Fluoreszenzauswertung mit der an sich bekannten Raman-Spektroskopie. Hierbei kann eine einzige Lichtquelle sowohl zur Fluoreszenzanregung als auch zur Rama-Spektroskopie verwendet werden. Die Anregung erfolgt dann vorzugsweise mit einem Laser im grünen Wellenlängenbereich mit einer Wellenlänge im Bereich vom 510nm-550nm. Für die Auswertung reicht dann auch ein einziges Spektrometer, da die im Rahmen der Raman-Spektroskopie ausgewerteten Stokes-Linien mit der Fluoreszenzantowrt überlappen. Dies wäre ein sehr kostengünstiges Dual-Messverfahren, bei dem neben einer Verifizierung der Fleischqualität auch die Fleischart (Pferd, Schwein, etc.) geprüft werden könnte und das mit nur einem einzigen Messgerät.

Die Erfindung beruht auch auf der Erkenntnis, dass die störenden Fehlmessungen bei dem eingangs genannten bekannten Messgerät dadurch verursacht werden können, dass die Messung an einer lokal begrenzten Unregelmäßigkeit auf der Oberfläche des Fleischs erfolgt, beispielsweise im Bereich einer Fettfaser oder eines Knochens.

Die Erfindung umfasst deshalb auch die allgemeine technische Lehre, die Messung nicht nur an einem einzigen Messpunkt auf dem zu überwachenden Lebensmittel durchzuführen, sondern an mehreren Messpunkten, wobei die Messpunkte voneinander beabstandet sind. Beispielsweise kann das erfindungsgemäße Messgerät vier verschiedene Messpunkte aufweisen, jedoch ist im Rahmen der Erfindung auch eine größere Anzahl (z.B. 5, 6, 7, 8 oder n>8) oder eine kleinere Anzahl (z.B. 2, 3) von Messpunkten möglich.

Schließlich umfasst die Erfindung auch die neuartige Verwendung eines solchen Messgeräts zum Messen von Stoffwechselprodukten von Bakterien auf einem Lebensmittel, das zum Verzehr bestimmt ist, insbesondere auf Fleisch.

Hierbei kann das erfindungsgemäße Messgerät auch an einer Fertigungsstraße eingesetzt werden, auf der Lebensmittel bearbeitet wird, wobei das Lebensmittel (z.B. Fleisch) entlang der Fertigungsstraße transportiert wird und dort verschiedenen Bearbeitungsschritten (z.B. Aufnehmen, Schneiden, Portionieren, Wiegen, Vermessen, Zubereiten, Anrichten und/oder Verpacken) unterworfen wird. Entlang der Fertigungsstraße erfolgt also mittels des erfindungsgemäßen Messgeräts vorzugsweise auch eine Messung des Frischezustands des Fleischs bzw. des Lebensmittels. Bei einer Verpackung des Lebensmittels mittels durchsichtiger Frischhaltefolie kann der Frischezustand auch durch die Verpackung hindurch erfolgen.

Weiterhin ist zu erwähnen, dass die Erfindung nicht beschränkt ist auf die Vermessung von Fleisch. Vielmehr eignet sich das erfindungsgemäße Prinzip auch zur Vermessung von Oberflächenbelägen auf anderen Typen von Lebensmitteln, wie beispielsweise Fisch, Obst und Gemüse.

Ferner muss es sich bei dem Messobjekt nicht um ein Lebensmittel handeln. Das erfindungsgemäße Messgerät eignet sich vielmehr auch zum Messen von anderen Messobjekten, wie beispielsweise der Körperoberfläche eines lebenden Menschen, um beispielsweise Wunden beurteilen zu können. Das erfindungsgemäße Messgerät kann also beispielsweise auch als Wund-Scanner ausgebildet sein.

Andere vorteilhafte Weiterbildungen sind in den Unteransprüchen gekennzeichnet oder werden nachstehend zusammen mit der Beschreibung der bevorzugten Ausführungsbeispiele der Erfindung anhand der Figuren näher erläutert. Es zeigen:
- Figur 1: eine Perspektivansicht eines erfindungsgemäßen Messgeräts von schräg vorne,
- Figur 2: eine Frontansicht des Messkopfs des Messgeräts gemäß Figur 1,
- Figur 3: eine Perspektivansicht des Messgeräts aus den Figuren 1 und 2 von schräg hinten,
- Figur 4: eine schematische Darstellung des erfindungsgemäßen Messgeräts,
- Figur 5: ein Spektraldiagramm mit den Spektren der Fluoreszenzstrahlung zu verschiedenen Zeitpunkten während der Lagerung des Fleischs,
- Figur 6: eine vereinfachte Darstellung zur Bestimmung des Ausgangssignals durch eine Fuzzy-Logik, sowie
- Figur 7: eine vereinfachte Darstellung einer Fertigungsstraße in der lebensmittelverarbeitenden Industrie mit dem erfindungsgemäßen Messgerät zur Bestimmung des Frischezustands des verarbeiteten Fleischs.

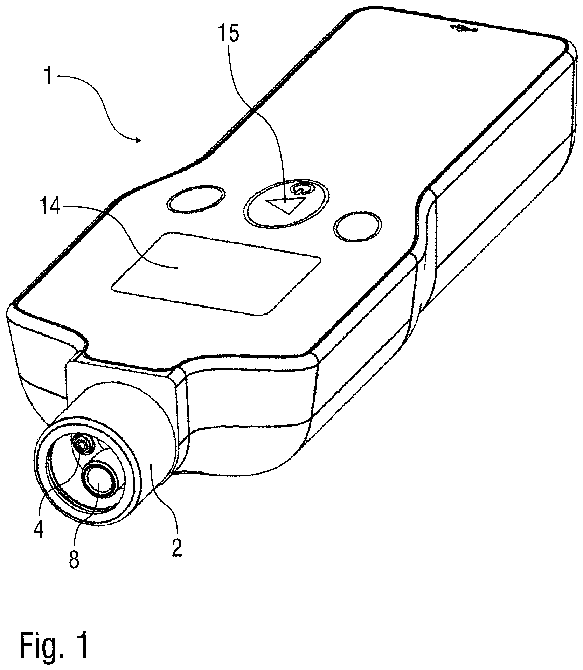

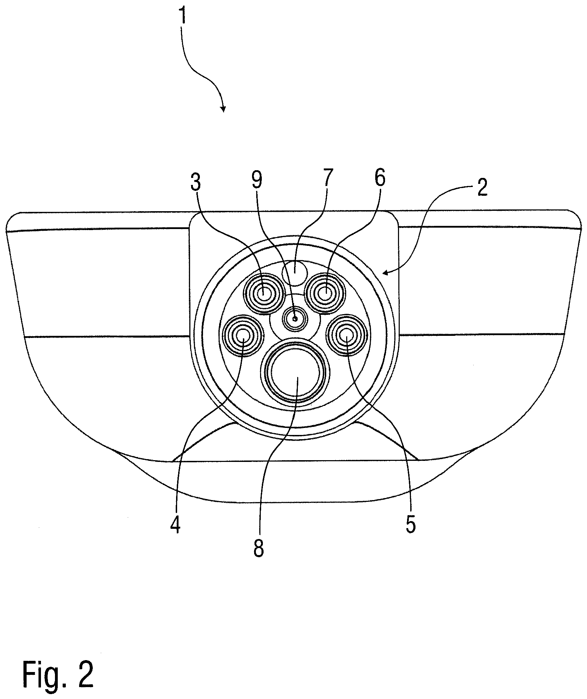

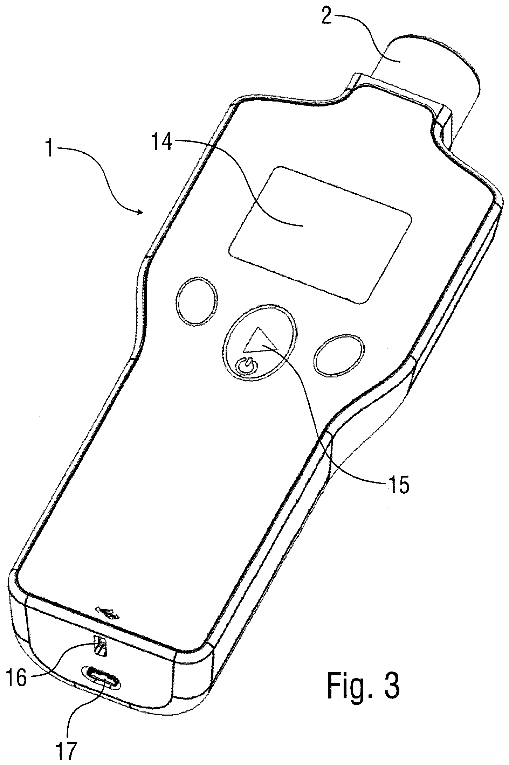

Die Figuren 1 bis 4 zeigen ein erfindungsgemäßes Messgerät 1 zur Messung des Frischezustands von Fleisch durch Anregung und Messung von Fluoreszenzstrahlung, die von bakteriellen Stoffwechselprodukten (Porphyrinen) auf dem Fleisch abgegeben wird.

Hierzu weist das Messgerät 1 einen Messkopf 2 aus V4A-Stahl auf, wobei auf den Messkopf 2 eine durchsichtige Messkappe aufgesetzt werden kann, um eine Verschmutzung durch das Lebensmittel zu vermeiden. Die Anregung der Fluoreszenzstrahlung in dem Oberflächenbelag auf dem Fleisch und die Messung der von dem Oberflächenbelag auf dem Fleisch ausgehenden Fluoreszenzstrahlung erfolgt hierbei durch die durchsichtige Messkappe hindurch.

Diese Messkappe kann auch einen spektralen optischen ph-Indikator-Mikropunkt integriert haben. Somit kann über das Spektrum auch noch optisch der ph-Wert ermittelt werden.

Der Messkopf 2 enthält vier Laserdioden 3-6, die zur Anregung der Fluoreszenzstrahlung ultraviolettes Licht abstrahlen.

Weiterhin enthält der Messkopf 2 ein Pyrometer 7 zur berührungslosen Temperaturmessung der Oberflächentemperatur des Oberflächenbelags auf dem Fleisch.

Darüber hinaus enthält der Messkopf 2 noch ein Kolorimeter 8 mit einer kalibrierten Leuchtdiode zur kolorimetrischen spektralen Messung, d.h. zur Farbmessung des Oberflächenbelags ohne die Fluoreszenzanregung.

Schließlich enthält der Messkopf 2 noch einen Kollektor 9 einer Lichtleitfaser, wobei der Kollektor 9 die von dem Oberflächenbelag auf dem Fleisch abgestrahlte Fluoreszenzstrahlung erfasst und über die Lichtleitfaser zu einem entsprechenden optischen Sensor 10 weiterleitet.

Die vier Laserdioden 3-6 beleuchten die Oberfläche des Fleischs an vier räumlich getrennten Messpunkten M1-M4, so dass an den vier Messpunkten M1-M4 jeweils Fluoreszenzstrahlung erzeugt wird, die dann von dem Kollektor 9 erfasst wird. Die Messung an den vier verschiedenen Messpunkten M1-M4 bietet den Vorteil, dass lokale Unregelmäßigkeiten (z.B. durch Fetteinlagerungen oder Knochen) bei der Messung kompensiert werden können und deshalb nicht zu Fehlmessungen führen.

Darüber hinaus kann das erfindungsgemäße Messgerät 1 ein pH-Meter 11 aufweisen, das den pH-Wert des Oberflächenbelags auf dem Fleisch misst.

Weiterhin kann das Messgerät ein Ohmmeter 12 aufweisen, das den elektrischen Oberflächenwiderstand des Oberflächenbelags auf dem Fleisch misst.

Schließlich kann das Messgerät 1 noch ein Messglied 13 aufweisen, welches das zeitliche Abklingverhalten der von dem Oberflächenbelag auf dem Fleisch abgestrahlten Fluoreszenzstrahlung erfasst.

An seiner Außenseite weist das erfindungsgemäße Messgerät 1 ein Display 14 auf, auf dem unter anderem das Messergebnis ausgegeben wird.

Darüber hinaus weist das Messgerät 1 an seiner Oberseite eine Folientastatur 15 auf, über die Benutzereingaben vorgenommen werden können.

Weiterhin sind in dem Gehäuse des Messgeräts 1 noch ein sogenanntes Kensington-Schloss 16 und eine USB-Schnittstelle 17 angeordnet.

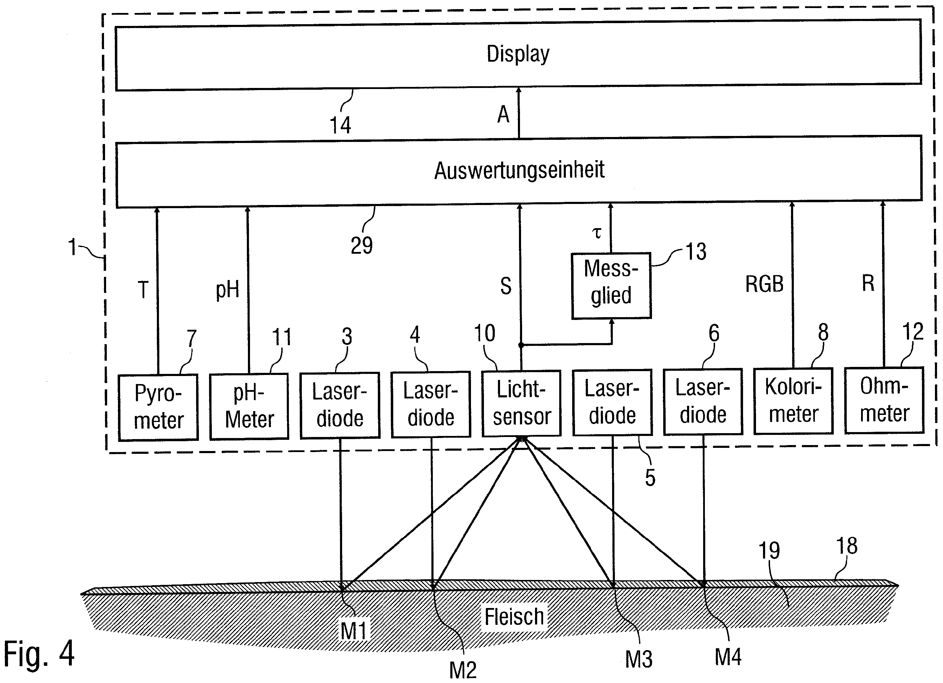

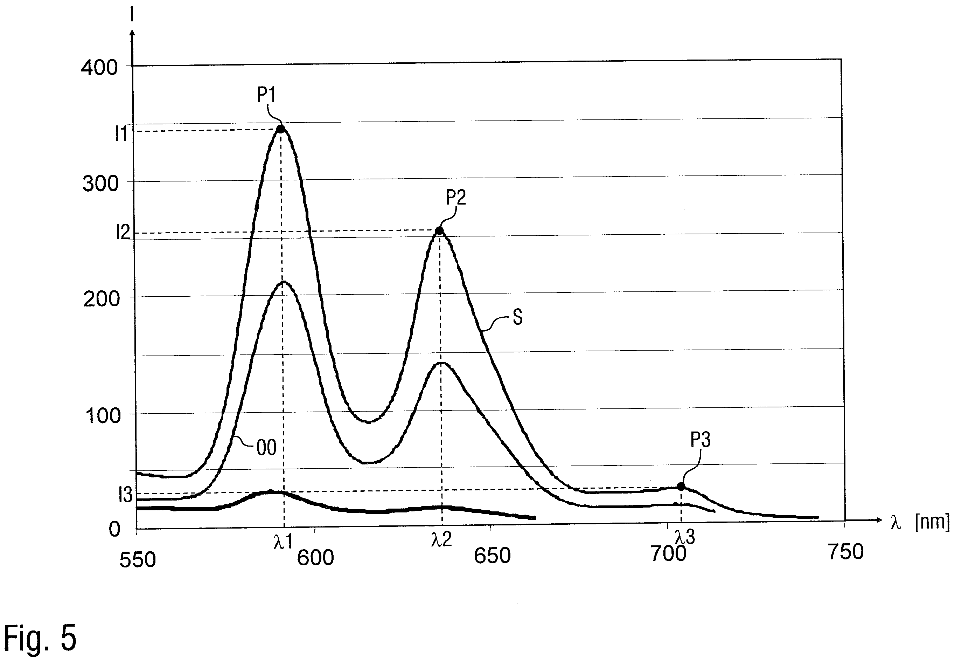

Im Folgenden wird nun unter Bezugnahme auf die Figuren 4 und 5 die Betriebsweise des erfindungsgemäßen Messgeräts 1 beschrieben.

Die Laserdioden 3-6 beleuchten jeweils einen der Messpunkte M1-M4 auf einem Oberflächenbelag 18 eines zu prüfenden Fleischs 19. Die in dem Oberflächenbelag 18 enthaltenen bakteriellen Stoffwechselprodukte (Porphyrine) erzeugen dann jeweils Fluoreszenzstrahlung, die von dem optischen Sensor 10 über den Kollektor 9 gemessen wird. Der optische Sensor 10 gibt dann ein entsprechendes Wellenlängenspektrum S an eine Auswertungseinheit 20.

Die Auswertungseinheit 20 ermittelt dann von dem gemessenen Spektrum S jeweils die Peaks P1, P2 und P3. Bei den einzelnen Peaks P1, P2, P3 des gemessenen Fluoreszenzspektrums S wird jeweils die Intensität I1, 12 bzw. 13 und die Wellenlänge λ1, λ2 bzw. λ3 gemessen.

Anschließend berechnet die Auswertungseinheit 20 die Intensitätsverhältnisse V1=I1/I2, V2=I1/I3 und V3=I2/I3. Die Intensitätsverhältnisse V1, V2 und V3 werden dann nachfolgend als charakteristische Größen zur Qualifizierung des gemessenen Fluoreszenzspektrums S herangezogen.

Weiterhin ermittelt die Auswertungseinheit 20 für jeden der Peaks P1, P2, P3 des gemessenen Fluoreszenzspektrums die jeweilige Wellenlänge λ1, λ2 bzw. λ3. Für jeden der Peaks P1, P2, P3 wird dann die Wellenlängenabweichung berechnet zwischen der gemessenen Wellenlänge λ1, λ2 bzw. λ3 einerseits und vorgegebenen charakteristischen Wellenlängen λ1_{REF}, λ2_{REF} bzw. λ3_{REF} andererseits, wobei diese charakteristischen Wellenlängen λ1_{REF}, λ2_{REF} bzw. λ3_{REF} für die Fluoreszenzstrahlung von Porphyrinen charakteristisch sind. Die auf diese Weise ermittelten Wellenlängenabweichungen Δλ1=λ1-λ1_{REF}, Δλ2=λ2λA2_{REF} bzw. Δλ3=λ3-λ3_{REF} werden dann nachfolgend für die Qualifizierung des Fluoreszenzspektrums S herangezogen.

Darüber hinaus berücksichtigt die Auswertungseinheit 20 noch eine Temperatur T, die von dem Pyrometer 7 gemessen wird, einen Oberflächenwiderstand R, der von dem Ohmmeter 12 gemessen wird, einen Farbwert RGB, der von dem Kolorimeter 8 gemessen wird sowie das zeitliche Abklingverhalten, das von dem Messglied 13 gemessen wird und in Form einer Zeitkonstante τ an die Auswertungseinheit 20 übertragen wird.

Die Auswertungseinheit 20 gibt dann ein entsprechendes Auswertungssignal A an das Display 14, wobei das Ausgangssignal A den Frischezustand des Fleischs wiedergibt.

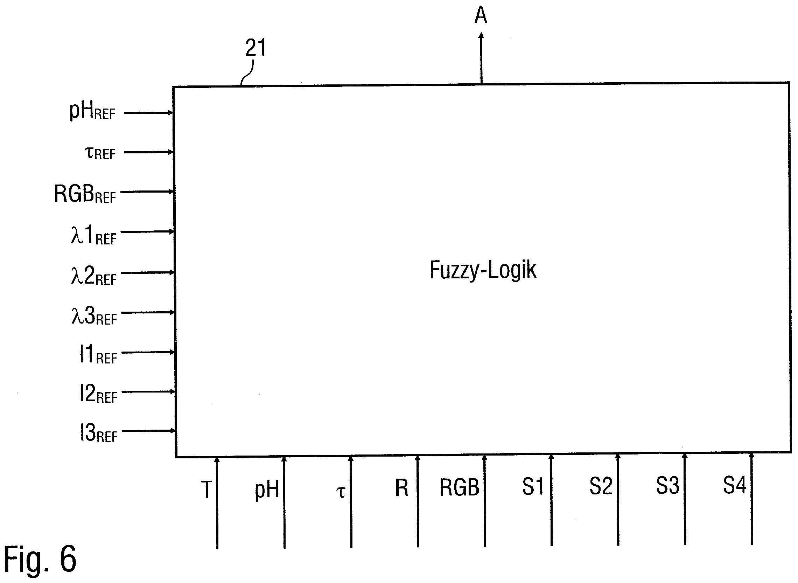

Figur 6 zeigt eine Fuzzy-Logik 21 zur Bestimmung des Ausgangssignals A in Abhängigkeit von den vorstehend beschriebenen Eingangsgrößen. Die Funktionsweise einer derartigen Fuzzy-Logik ist an sich aus dem Stand der Technik bekannt und muss deshalb nicht näher beschrieben werden.

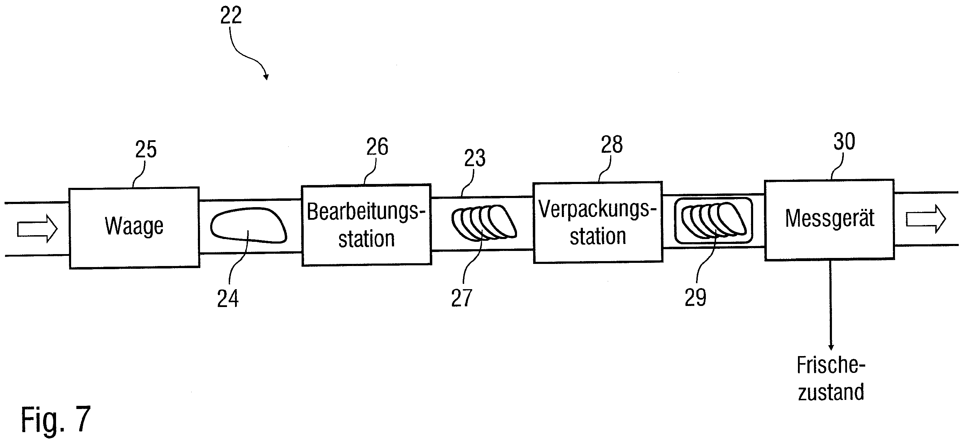

Figur 7 zeigt schließlich ein exemplarisches Einsatzgebiet der Erfindung in einer Fertigungsstraße 22 zur industriellen Lebensmittelverarbeitung.

Die Fertigungsstraße 22 umfasst ein Förderband 23, auf dem Fleisch 24 in Pfeilrichtung transportiert wird.

Am Eingang der Fertigungsstraße 22 befindet sich eine Waage 25, welche das Fleisch 24 wiegt.

In Förderrichtung hinter der Waage 25 befindet sich eine Bearbeitungsstation 26, die das Fleisch 24 bearbeitet. In diesem Ausführungsbeispiel handelt es sich bei der Bearbeitungsstation 26 um eine Schneideinrichtung, die das Fleisch 24 in mehrere Scheiben 27 aufschneidet.

In Förderrichtung hinter der Bearbeitungsstation 26 befindet sich eine Verpackungsstation 28, welche die Fleischscheiben 27 in eine Klarsichtverpackung 29 verpackt.

In Förderrichtung hinter der Verpackungsstation 28 befindet sich dann ein erfindungsgemäßes Messgerät 30, das durch die Klarsichtverpackung 29 hindurch den Frischezustand des verpackten Fleischs misst, wie bereits vorstehend beschrieben worden ist.

Die Erfindung ist nicht auf die vorstehend beschriebenen bevorzugten Ausführungsbeispiele beschränkt. Vielmehr ist eine Vielzahl von Varianten und Abwandlungen möglich, die ebenfalls von dem Erfindungsgedanken Gebrauch machen und deshalb in den Schutzbereich fallen. Insbesondere beansprucht die Erfindung auch Schutz für den Gegenstand und die Merkmale der Unteransprüche unabhängig von den in Bezug genommenen Ansprüchen. Beispielsweise genießen die Unteransprüche auch Schutz ohne das kennzeichnende Merkmal des Hauptanspruchs.

### Bezugszeichenliste:

- A: Ausgangssignal der Auswertungseinheit
- M1-M4: Messpunkte
- S: Fluoreszenzspektrum
- P1: Peak des Fluoreszenzspektrums
- P2: Peak des Fluoreszenzspektrums
- P3: Peak des Fluoreszenzspektrums
- V1: Intensitätsverhältnis I1/I2
- V2: Intensitätsverhältnis I1/I3
- V3: Intensitätsverhältnis 12/13
- λ1: Wellenlänge des Peaks P1
- λ2: Wellenlänge des Peaks P2
- λ3: Wellenlänge des Peaks P3
- T: Temperatur
- R: Oberflächenwiderstand
- RGB: Farbwert
- pH: pH-Wert
- τ: Zeitkonstante des Abklingverhalten der Fluoreszenzstrahlung
- 1: Messgerät
- 2: Messkopf
- 3: Laserdiode
- 4: Laserdiode
- 5: Laserdiode
- 6: Laserdiode
- 7: Pyrometer
- 8: Kolorimeter
- 9: Kollektor
- 10: Optischer Sensor
- 11: pH-Meter
- 12: Ohmmeter
- 13: Messglied
- 14: Display
- 15: Folientastatur
- 16: Kensington-Schloss
- 17: USB-Schnittstelle
- 18: Oberflächenbelag
- 19: Fleisch
- 20: Auswertungseinheit
- 21: Fuzzy-Logik
- 22: Fertigungsstraße
- 23: Förderband
- 24: Fleisch
- 25: Waage
- 26: Bearbeitungsstation
- 27: Scheiben
- 28: Verpackungsstation
- 29: Klarsichtverpackung
- 30: Messgerät

## Patentansprüche

1. Messgerät (1; 30) zum Messen eines Oberflächenbelags (18) auf einem Messobjekt, nämlich auf einem Lebensmittel (19; 24), insbesondere zum Messen von Stoffwechselprodukten von Bakterien auf Fleisch, das zum Verzehr bestimmt ist, mit
a) mehreren Anregungsquellen (3-6) zur Photolumineszenzanregung des Oberflächenbelags (18) auf dem zu überwachenden Messobjekt (19; 24), so dass der Oberflächenbelag (18) eine Photolumineszenzstrahlung abgibt,
a1) wobei die Anregungsquellen (3-6) Lichtquellen (3-6) sind, nämlich Laserdioden (3-6), und
a2) die Lichtquellen (3-6) zur Photolumineszenzanregung Licht mit einem Spektrum von 350nm-550nm emittieren, insbesondere mit einer Wellenlänge von 405nm, und
b) mindestens einem optischen Sensor (10) zur Erfassung der Photolumineszenzstrahlung, die von dem Oberflächenbelag (18) abgegeben wird, wobei
b1) das Messgerät (1; 30) die Photolumineszenzstrahlung des Oberflächenbelags (18) an mehreren voneinander beabstandeten Messpunkten (M1-M4) auf dem zu überwachenden Messobjekt (19; 24) misst, insbesondere an vier Messpunkten (M1-M4),
b2) der optische Sensor (10) die Photolumineszenzstrahlung von allen Messpunkten (M1-M4) erfasst,
b3) die Messpunkte (M1-M4) um die optische Achse des optischen Sensors (10) verteilt angeordnet sind,
b4) der optische Sensor (10) einen so großen Messwinkel aufweist, dass alle Messpunkte (M1-M4) innerhalb des Messwinkels liegen, so dass der optische Sensor (10) die Photolumineszenzstrahlung von allen Messpunkten (M1-M4) messen kann, und
b5) der optische Sensor (10) ein Spektralphotometer ist, das ein Wellenlängenspektrum der Photolumineszenzstrahlung des Oberflächenbelags (18) misst, und mit
c) einer Auswertungseinheit (20; 21) zur Qualifizierung und/oder Quantifizierung des Oberflächenbelags (18) und/oder des Lebensmittels (19; 24) in Abhängigkeit von dem Wellenlängenspektrum der Photolumineszenzstrahlung des Oberflächenbelags (18) und zur Erzeugung eines entsprechenden Ausgangssignals (A).

2. Messgerät (1; 30) nach Anspruch 1, **dadurch gekennzeichnet, dass** die von dem Oberflächenbelag (18) abgegebene Photolumineszenzstrahlung über einen Lichtleiter zu dem optischen Sensor (10) geleitet wird.

3. Messgerät (1; 30) nach einem der vorhergehenden Ansprüche, **gekennzeichnet durch** ein Pyrometer (7) zur berührungslosen Temperaturmessung der Oberflächentemperatur des Oberflächenbelags (18) auf dem zu überwachenden Messobjekt (19; 24).

4. Messgerät (1; 30) nach einem der vorhergehenden Ansprüche, **gekennzeichnet durch** ein Kolorimeter (8) zur Messung der Farbe (RGB) des Oberflächenbelags (18) ohne die Photolumineszenzanregung, insbesondere mittels einer Leuchtdiode.

5. Messgerät (1; 30) nach einem der vorhergehenden Ansprüche, **gekennzeichnet durch** ein Messglied (13) zur Messung des zeitlichen Abklingverhaltens (τ) der Photolumineszenzstrahlung des Oberflächenbelags (18).

6. Messgerät (1; 30) nach einem der vorhergehenden Ansprüche, **dadurch gekennzeichnet, dass** das Ausgangssignal (A) mindestens eine der folgenden Eigenschaften des Lebensmittels (19; 24) wiedergibt:
a) Keimzahl von Stoffwechselprodukten von Bakterien in dem Oberflächenbelag (18),
b) Keimzahl von von Porphyrinen, insbesondere von Protoporphyrin IX,
c) Typ des Lebensmittels (19; 24) aus einer Gruppe von Fleisch, Fisch, Gemüse und/oder Obst,
d) Typ des Fleischs aus einer Gruppe von Schweinfleisch, Rindfleisch, Geflügelfleisch, Lammfleisch, Wildfleisch, Pferdefleisch und/oder Hundefleisch,
e) Verzehrbarkeit des Lebensmittels (19; 24) in Abhängigkeit vom Frischezustand des Lebensmittels (19; 24).

7. Messgerät (1; 30) nach einem der vorhergehenden Ansprüche, **dadurch gekennzeichnet, dass** die Auswertungseinheit den Oberflächenbelag (18) in Abhängigkeit von mindestens einer der folgenden Größen qualifiziert und/oder quantifiziert, insbesondere mittels einer Fuzzy-Logikeinheit:
a) einem Gesamtwert, der die Intensitätspektren (11-13) der Photolumineszenzstrahlungen an allen Messpunkten (M1-M4) wiedergibt,
b) den von dem optischen Sensor (10) gemessenen Intensitätsspektren (11-13) der Photolumineszenzstrahlung an den einzelnen Messpunkten (M1-M4),
c) der von dem Pyrometer (7) gemessenen Oberflächentemperatur (T),
d) der von dem Kolorimeter (8) gemessenen Farbe (RGB),
e) dem zeitlichen Abklingverhalten (τ) der Photolumineszenzstrahlung.

8. Messgerät (1; 30) nach einem der vorhergehenden Ansprüche, **dadurch gekennzeichnet,**
a) **dass** das Messgerät (1; 30) ein tragbares Handgerät ist, und
b) **dass** das Messgerät (1; 30) zur Stromversorgung eine Batterie enthält, insbesondere eine wiederaufladbare Batterie, und
c) **dass** das Messgerät (1; 30) zur Anzeige des Ausgangssignals (A) eine optische Anzeige (14) aufweist.

9. Messgerät (1; 30) nach einem der vorhergehenden Ansprüche, **dadurch gekennzeichnet, dass** das Messgerät (1; 30) eine Messdauer aufweist, die kleiner ist als 10s.

10. Messgerät (1; 30) nach einem der vorhergehenden Ansprüche, **dadurch gekennzeichnet, dass** das Messgerät (1; 30) mindestens eine Datenschnittstelle (17) aufweist zur Konfigurierung des Messgeräts (1; 30) und/oder zur Ausgabe von Messdaten, wobei die Datenschnittstelle vorzugsweise eine USB-Schnittstelle (17), eine Bluetooth-Schnittstelle und/oder eine RFID-Schnittstelle aufweist.

11. Messgerät (1; 30) nach einem der vorhergehenden Ansprüche, **dadurch gekennzeichnet, dass** das Messgerät (1; 30) eine durchsichtige und auswechselbare Kappe aufweist, wobei die Anregung der Lumineszenzstrahlung und die Messung der Lumineszenzstrahlung durch die Kappe hindurch erfolgt.

12. Messgerät (1; 30) nach einem der vorhergehenden Ansprüche, **dadurch gekennzeichnet, dass** jedem Messpunkt (M1-M4) jeweils eine Anregungsquelle (3-6) zugeordnet ist, so dass die Photolumineszenzanregung an jedem Messpunkt (M1-M4) jeweils durch die diesem Messpunkt (M1-M4) zugeordnete Anregungsquelle (3-6) erfolgt.

13. Messgerät (1; 30) nach einem der Ansprüche 2 bis 12, **gekennzeichnet durch** einen Kollektor (9) des Lichtleiters, wobei der Kollektor (9) die von dem Oberflächenbelag auf dem Lebensmittel abgestrahlte Photolumineszenzstrahlung erfasst und über den Lichtleiter zu dem mindestens einen optischen Sensor (10) weiterleitet.

14. Fertigungsstraße (22) zur Bearbeitung von Lebensmitteln (24), mit
a) mehreren Bearbeitungsstationen (25, 26, 28), die entlang der Fertigungsstraße (22) hintereinander angeordnet sind und die das Lebensmittel (19; 24) aufnehmen, schneiden, portionieren, wiegen, vermessen, zubereiten, anrichten und/oder verpacken, und
b) einem Messgerät nach einem der vorgehenden Ansprüche zur Vermessung des Lebensmittels (19; 24) auf der Fertigungsstraße (22), insbesondere nach der Verpackung des Lebensmittels (19; 24) durch die Verpackung (29) hindurch.
